# EUROPEAN PATENT APPLICATION

(11) **EP 4 138 378 A1**
(43) Date of publication of application: **22.02.2023**
(21) Application number: 21787656.4
(22) Date of filing: 04.02.2021
(51) Int. Cl.: H04M 3/42, H04M 11/00, G06F 16/909, G06F 13/00

(54) **INFORMATION PROCESSING SYSTEM, INFORMATION PROCESSING METHOD, AND COMPUTER PROGRAM**

(30) Priority: 13.04.2020 JP 2020071397; 20.05.2020 JP 2020088305
(71) Applicant: IoT-EX Inc., Chiyoda-ku Tokyo 101-0043 (JP)
(72) Inventor: MATSUMURA Jun, Tokyo 101-0043 (JP)
(74) Representative: REHBERG HÜPPE + PARTNER
(86) International application number: PCT/JP2021/004039
(87) International publication number: WO 2021/210247

(57) **Abstract**

An information processing system, an information processing method, and a computer program capable of easily and reliably identifying a behavioral history of an infected person and extracting close contacts are provided.

According to an embodiment of the present disclosure, there is provided an information processing system including a first information processing device having a function of transmitting a signal, a second information processing device having a function of receiving the signal, and a server device capable of being connected to the second information processing device via the Internet, wherein the server device stores time information, intensity information, first identification information, and second identification information received from the second information processing device in association with each other and extracts one or more pieces of other first identification information stored in association with specific second identification information on the basis of the specific second identification information stored in association with specific first identification information when the designation of the specific first identification information of a specific first information processing device has been received.

## Description

### [TECHNICAL FIELD1

The present invention relates to an information processing system, an information processing method, and a computer program.

### [BACKGROUND ART]

When a person suffering from an infectious disease such as the new coronavirus (COVID-19) (hereinafter referred to as an "infected person") has been identified, it is necessary to know a behavioral history of the infected person to identify other highly infectious persons associated with the infected person (hereinafter referred to as "close contacts").

The close contacts described here are generally people who have been in contact with a "patient (confirmed case)" at a distance where hand contact or a face-to-face conversation was possible without a necessary infection prevention countermeasure.

At present, this behavioral history is generally obtained by conducting interviews with infected people and is uncertain.

Recently, an application for identifying close contacts on the basis of information when people become infected by recording information of other people nearby on their own smartphones using short-range wireless communication functions between smartphones has been developed.

### [CITATION LIST]

### [NON-PATENT LITERATUREI

### [NON-PATENT LITERATURE 1]

"Singapore launches app to track coronavirus infections with government development," Nihon Keizai Shimbun electronic version, retrieved on April 2, 2020 <URL: https://www.nikkei.com/article/DGXMZO57056430Q0A320C2FF8000/>

### [SUMMARY OF INVENTION]

### [TECHNICAL PROBLEM]

However, according to such technology, because information of others is recorded in individual smartphones, there is a problem that management by the behavioral history tracking team is not easy.

Therefore, an objective of the present disclosure is to provide an information processing system, an information processing method, and a computer program capable of easily and reliably identifying a behavioral history of an infected person and extracting close contacts.

### [SOLUTION TO PROBLEM]

According to an embodiment of the present disclosure, there is provided an information processing system including a first information processing device having a function of transmitting a signal, a second information processing device having a function of receiving the signal, and a server device capable of being connected to the second information processing device via the Internet, wherein the first information processing device transmits a signal including first identification information of the first information processing device, wherein the second information processing device receives the signal transmitted from the first information processing device and transmits time information about the time at which the signal was received, intensity information based on a reception intensity of the signal, and the first identification information to the server device together with second identification information of the second information processing device, wherein the server device stores the time information, the intensity information, the first identification information, and the second identification information received from the second information processing device in association with each other, and wherein the server device extracts one or more pieces of other first identification information stored in association with specific second identification information on the basis of the specific second identification information stored in association with specific first identification information when the designation of the specific first identification information of a specific first information processing device has been received.

The server device can extract one or more pieces of other first identification information stored in association with specific time information on the basis of the specific time information about a specific time at which a signal including the specific second identification information stored in association with the specific first identification information and the specific first identification information was received when the designation of the specific first identification information has been received.

The server device can provide a notification of specific information to the first information processing device having the one or more pieces of the first identification information that are extracted when the designation of the specific first identification information has been received.

The second information processing device can be connected to the server device via the Internet, acquire environmental information of an installation location from a prescribed sensor, and transmit time information about the time at which the environmental information was received to the server device together with the environmental information, and the server device can store the environmental information and the time information received from the second information processing device in association with the second identification information.

The server device can extract one or more pieces of the first identification information stored in association with the second identification information when the environmental information received from the second information processing device satisfies a specific condition.

The server device can provide a notification of caution information to at least one of first information processing devices having the one or more pieces of the first identification information extracted when the specific condition is satisfied.

The second information processing device can be an Internet of Things (IoT) router in an loT connection system including an loT hub implemented on a cloud and the loT router locally connected to the loT hub, the loT hub can have at least one of a first driver for connecting a private cloud and the loT hub to which a first device is connectable and a second driver for connecting a second device and the loT hub, and the loT router can have a third driver for connecting a third device and the loT router.

The server device can transmit information about an unlock key capable of unlocking a specific electronic lock only to the first information processing device corresponding to the first identification information received from the second information processing device.

According to an embodiment of the present disclosure, there is provided an information processing method to be executed by an information processing system including a first information processing device having a function of transmitting a signal, a second information processing device having a function of receiving the signal, and a server device capable of being connected to the second information processing device via the Internet, the information processing method including steps of: transmitting, by the first information processing device, a signal including first identification information of the first information processing device; receiving, by the second information processing device, the signal transmitted from the first information processing device; transmitting, by the second information processing device, time information about the time at which the signal was received, intensity information based on a reception intensity of the signal, and the first identification information to the server device together with second identification information of the second information processing device; storing, by the server device, the time information, the intensity information, the first identification information, and the second identification information received from the second information processing device in association with each other; receiving, by the server device, the designation of specific first identification information of a specific first information processing device; and extracting, by the server device, one or more pieces of other first identification information stored in association with specific second identification information on the basis of the specific second identification information stored in association with the specific first identification information when the designation has been received.

According to an embodiment of the present disclosure, there is provided a computer program to be executed in an information processing system including a first information processing device having a function of transmitting a signal, a second information processing device having a function of receiving the signal, and a server device capable of being connected to the second information processing device via the Internet, the computer program causing the second information processing device to implement functions of: receiving a signal including first identification information of the first information processing device transmitted from the first information processing device; and transmitting time information about the time at which the signal was received, intensity information based on a reception intensity of the signal, and the first identification information to the server device together with second identification information of the second information processing device, wherein the server device stores the time information, the intensity information, the first identification information, and the second identification information received from the second information processing device in association with each other and extracts one or more pieces of other first identification information stored in association with specific second identification information on the basis of the specific second identification information stored in association with specific first identification information when the designation of the specific first identification information of a specific first information processing device has been received.

### [ADVANTAGEOUS EFFECTS OF INVENTION]

According to the present disclosure, it is possible to provide an information processing system, an information processing method, and a computer program capable of easily and reliably identifying a behavioral history of an infected person and extracting close contacts.

### [BRIEF DESCRIPTION OF DRAWINGS]

In the following, the invention is further explained and described with respect to preferred exemplary embodiments illustrated in the drawings.
- **Fig. 1**: is a system configuration diagram showing an example of a configuration of an information processing system according to an embodiment of the present disclosure.
- **Fig. 2**: is a hardware configuration diagram showing a hardware configuration of a first information processing device according to the embodiment of the present disclosure.
- **Fig. 3**: is a functional configuration diagram showing a functional configuration of the first information processing device according to the embodiment of the present disclosure.
- **Fig. 4**: is a functional configuration diagram showing a functional configuration of a second information processing device according to the embodiment of the present disclosure.
- **Fig. 5**: is a functional configuration diagram showing the functional configuration of a server device according to the embodiment of the present disclosure.
- **Fig. 6**: is an image diagram showing an example of a data table stored in a storage unit of the server device according to the embodiment of the present disclosure.
- **Fig. 7**: is an image diagram showing an example of a data table stored in the storage unit of the server device according to the embodiment of the present disclosure.
- **Fig. 8**: is a functional configuration diagram showing another example of the functional configuration of the server device according to the embodiment of the present disclosure.
- **Fig. 9**: is a functional configuration diagram showing another example of the functional configuration of the second information processing device according to the embodiment of the present disclosure.
- **Fig. 10**: is a system configuration diagram showing an example of a configuration of an IoT connection system according to the embodiment of the present disclosure.
- **Fig. 11**: is an image diagram showing an example of a plurality of private clouds of the loT connection system according to the embodiment of the present disclosure.
- **Fig. 12**: is an image diagram showing an example of a plurality of first devices connected to the private cloud of the loT connection system according to the embodiment of the present disclosure.
- **Fig. 13**: is an image diagram showing an example of a flow of a service provided by the IoT connection system according to the embodiment of the present disclosure.
- **Fig. 14**: is a hardware configuration diagram showing a hardware configuration of an loT router according to the embodiment of the present disclosure.
- **Fig. 15**: is an image diagram showing an example of a flow of a service provided by the loT connection system according to the embodiment of the present disclosure.
- **Fig. 16**: is an image diagram for describing a connection interface of the loT connection system according to the embodiment of the present disclosure.
- **Fig. 17**: is an image diagram showing another example of the flow of the service provided by the loT connection system according to the embodiment of the present disclosure.
- **Fig. 18**: is an image diagram showing an example of a flow of a virtual driver function provided by the loT connection system according to the embodiment of the present disclosure.
- **Fig. 19**: is an image diagram showing another example of the flow of the virtual driver function provided by the IoT connection system according to the embodiment of the present disclosure.
- **Fig. 20**: is a flow diagram showing an example of a flow of an information processing method according to the embodiment of the present disclosure.
- **Fig. 21**: is a conceptual diagram showing another example of a configuration of the information processing system according to the embodiment of the present disclosure.

### [DESCRIPTION OF EMBODIMENTS]

Hereinafter, an embodiment of an information processing system of the present disclosure will be described with reference to the drawings.

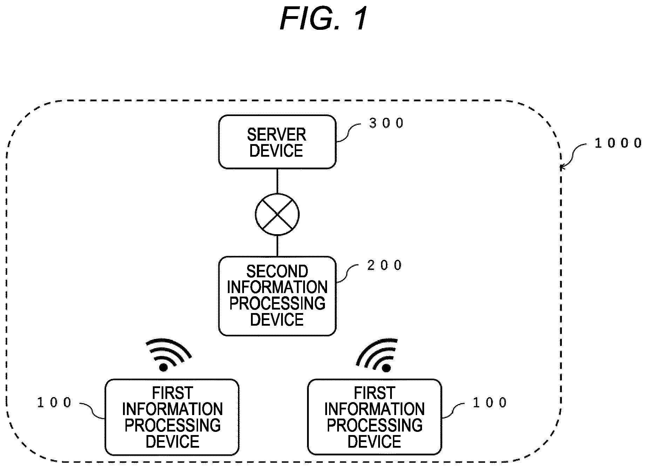

As shown in FIG. 1 as an example, an information processing system 1000 of the present disclosure includes a first information processing device 100, a second information processing device 200, and a server device 300.

The first information processing device 100 is a device having a function of transmitting a signal.

The function of transmitting the signal is, for example, a function in which information can be transmitted to a device capable of receiving the signal using wireless technologies such as Bluetooth (registered trademark) low energy (BLE) and Wi-Fi.

Although the first information processing device 100 is not particularly limited as long as it has the function of transmitting the above signal, the first information processing device 100 can be an information processing device such as a smartphone or a tablet as an example.

The function of transmitting the above signal can be implemented by the first information processing device 100 by installing a dedicated application.

The second information processing device 200 is a device having a function of receiving the above signal.

Although the second information processing device 200 is not particularly limited as long as it is a device having the function of receiving the above signal, the second information processing device 200 can be an information processing device such as a smartphone or a tablet as an example. Also, an loT router, which will be described below, may be used as the second information processing device 200.

The function of receiving the above signal can be implemented by the second information processing device 200 by installing a dedicated application.

The server device 300 is a device capable of being connected to the second information processing device 200 via the Internet.

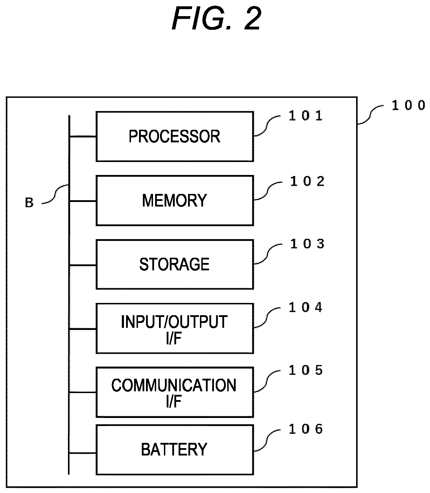

Here, a hardware configuration of the first information processing device 100 will be described using FIG. 2. The first information processing device 100 can include a processor 101, a memory 102, a storage 103, an input/output interface (input/output I/F) 104, and a communication interface (communication I/F) 105. The components are interconnected via a bus B.

The first information processing device 100 can implement the functions and methods described in the present embodiment through the cooperation of the processor 101, the memory 102, the storage 103, the input/output I/F 104, and the communication I/F 105.

The processor 101 executes functions and/or methods implemented by codes or instructions included in programs stored in the storage 103. The processor 201 may include, for example, a central processing unit (CPU), a micro processing unit (MPU), a graphics processing unit (GPU), a microprocessor, a processor core, a multiprocessor, an application-specific integrated circuit (ASIC), a field programmable gate array (FPGA), and the like and logic circuits (hardware) and dedicated circuits formed in integrated circuits (an integrated circuit (IC) chip and a large-scale integration (LSI) circuit) and the like may implement processes disclosed in the embodiments. Also, these circuits may be implemented by one or more integrated circuits and a plurality of processes shown in the embodiments may be implemented by one integrated circuit. Also, the LSI circuit may be referred to as a very large-scale integration (VLSI) circuit, a super-LSI circuit, an ultra-LSI circuit, or the like according to a difference in a degree of integration.

The memory 102 temporarily stores programs loaded from the storage 103 and provides a work area for the processor 101. The memory 102 also temporarily stores various types of data generated while the processor 101 is executing the program. The memory 102 includes, for example, a random access memory (RAM), a read only memory (ROM), and the like.

The storage 103 stores programs. The storage 103 includes, for example, a hard disk drive (HDD), a solid state drive (SSD), a flash memory, and the like.

The communication I/F 105 is implemented as hardware such as a network adapter, communication software, or a combination thereof and transmits and receives various types of data via a network. The communication may be performed by wire or wirelessly and any communication protocol may be used as long as mutual communication can be performed. The communication I/F 105 communicates with other information processing devices via the network. The communication I/F 105 transmits various types of data to other information processing devices in accordance with an instruction from the processor 101. Also, the communication I/F 105 also receives various types of data transmitted from other information processing devices and transmits the data to the processor 101.

The input/output I/F 104 includes an input device for inputting various types of operations to the first information processing device 100 and an output device for outputting a processing result after a process performed by the first information processing device 100. The input/output I/F 104 may be integrated with the input device and the output device or may be separated into the input device and the output device.

The input device is implemented by any one or a combination of all types of devices that can receive an input from the user and transmit information related to the input to the processor 101. The input device includes, for example, hardware keys of a touch panel, a touch display, and a keyboard, a pointing device such as a mouse, a camera (for an operation input via an image), and a microphone (for an operation input by voice).

The output device outputs a processing result of a process performed by the processor 101. The output device includes, for example, a touch panel, a speaker, and the like.

The second information processing device 200 and the server device 300 can also have the same hardware configuration as the first information processing device 100.

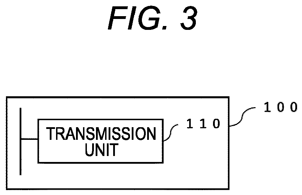

The first information processing device 100 in the present disclosure has a transmission unit 110 as shown in FIG. 3.

The transmission unit 110 transmits a signal including the first identification information of the first information processing device 100.

The first identification information is not particularly limited as long as it is identification information for identifying the first information processing device 100. Although a unique device identifier (UDID), a universally unique identifier (UUID), or the like can be used as an example of the first identification information, it is preferable to use the UUID as the first identification information from the viewpoint of privacy and security.

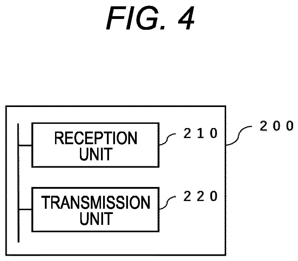

The second information processing device 200 has a reception unit 210 and a transmission unit 220, as shown in FIG. 4.

The reception unit 210 receives a signal transmitted from the first information processing device 100.

The transmission unit 220 transmits time information, intensity information, and first identification information to the server device 300 together with second identification information of the second information processing device 200.

The time information is information about the time at which the above signal was received. This time information can include a date and time and the date and time may include hours, minutes, and seconds.

The intensity information is information based on a reception intensity of the signal. The information about the reception intensity can include a reference intensity in addition to a radio wave intensity that is an intensity of received radio waves. It is possible to calculate an estimated distance between the first information processing device 100 and the second information processing device 200 from the above-described information and this estimated distance may be used as information based on the reception intensity. The calculation of the estimated distance may be performed by the second information processing device 200 or may be performed by the server device 300.

The second identification information is not particularly limited as long as it is identification information for identifying the second information processing device 200. Although a unique device identifier (UDID), a universally unique identifier (UUID), or the like can be used as an example of the second identification information as in the first identification information, the UUID is preferably used as the second identification information from the viewpoint of privacy and security. This UUID is a numerical value issued by the dedicated application described above and may not include personal information.

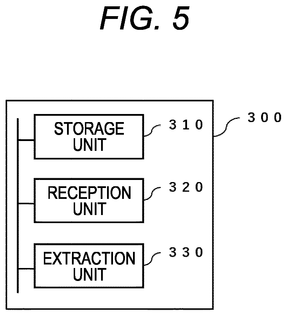

The server device 300 has a storage unit 310, a reception unit 320, and an extraction unit 330, as shown in FIG. 5.

The storage unit 310 stores the time information, the intensity information, the first identification information, and the second identification information received from the second information processing device 200 in association with each other.

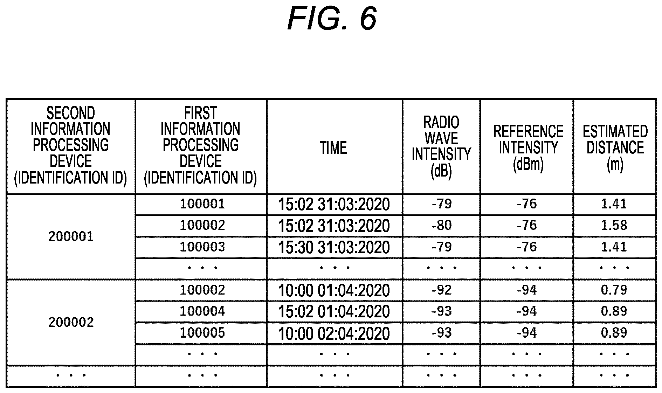

FIG. 6 shows an example of a data table stored in the storage unit 310. As shown in FIG. 6, the second information processing device 200 stores a reception time and a radio wave intensity of the received signal with the first information processing device 100 having transmitted the received signal.

Although only one piece of time information is shown for the first identification information in FIG. 6, the time information can be stored at prescribed intervals from the time when the signal is first received to the time when no signal is received.

The reception unit 320 receives the designation of specific first identification information of a specific first information processing device 100.

It is assumed that the above designation is performed via a prescribed information processing terminal by a user who uses the information processing system of the present disclosure.

The specific first information processing device 100 can be a portable terminal such as a smartphone owned by an infected person.

In this case, an example of a user who performs the above designation is a person who tracks a behavioral history of an infected person, such as a person in charge of a public health center or a medical worker, but the user is not limited to these persons.

For example, the infected person himself/herself can also transmit information indicating that he or she has been infected with an infectious disease from the first information processing device 100 to the server device 300 and designate specific first identification information of his or her own specific first information processing device 100A.

When the reception unit 320 has received the designation of the specific first identification information of the specific first information processing device 100, the extraction unit 330 extracts one or more pieces of other first identification information stored in association with the specific second identification information on the basis of the specific second identification stored in association with the specific first identification information.

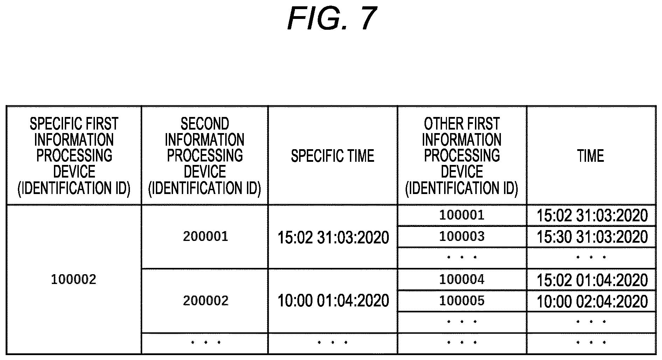

FIG. 7 shows an example in which an image of the other first identification information extracted when the specific first identification information of the specific first information processing device 100A is "100002" in FIG. 6 is shown.

Also, the extraction unit 330 may extract other second identification information on the basis of a filter condition based on time information and/or intensity information. Details will be described below.

As described above, according to the information processing system of the present disclosure, it is possible to easily and reliably acquire a behavioral history indicating when and where a person having a specific first information processing device was and it is possible to easily and reliably identify people who were in the same place as the person. That is, the information processing system in the present disclosure improves traceability of infected persons.

For example, if the second information processing device 200 is installed in a room in the company, information such as which room an infected person stayed in and for how long and who the close contacts were can be quickly extracted at the time of occurrent of the infected person.

By quickly identifying the close contacts, it is possible to quickly take the effective countermeasure such as placing only the close contacts on standby at home instead of all those who entered or left the company. As a result, it is possible to avoid a situation such as the complete closure of the office and it will also be a business continuity plan (BCP) countermeasure.

When the designation of the specific first identification information has been received, the extraction unit 330 can extract one or more pieces of other first identification information stored in association with specific time information on the basis of the specific time information about a specific time at which a signal including the specific second identification information stored in association with the specific first identification information and the specific first identification information has been received.

That is, the extraction unit 330 can extract other second identification information on the basis of a filter condition based on the time information.

The specific time information about the specific time can be, for example, all times stored at prescribed intervals from the time at which the specific first information processing device received the signal for the first time to the time at which no signal was received.

Alternatively, the specific time information about the specific time can include a staying period calculated on the basis of the time at which the specific first information processing device received the signal for the first time and a period of time from the time at which the specific first information processing device received the signal for the first time to the time at which no signal was received as another example.

The time information associated with the specific time information can be the time set on the basis of the specific time information and at which there is a high possibility of close contact with the infected person.

Specifically, in addition to those who were in the same place and time as the infected person, those who were in the same place as the infected person until a prescribed period of time has elapsed after the infected person disappeared can be extracted as close contacts.

Further, the extraction unit 330 may extract other second identification information on the basis of a filter condition based on intensity information.

That is, when an infected person is estimated to be in a room where the second information processing device 200 is installed, a person who is estimated to be outside of the room on the basis of the intensity information may not be extracted as a close contact.

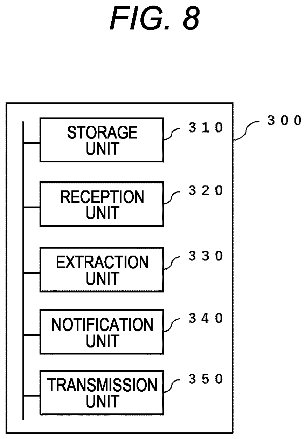

The server device 300 can further have a notification unit 340, as shown in FIG. 8.

The notification unit 340 provides a notification of specific information to the first information processing device 100 having one or more pieces of first identification information extracted when the designation of specific first identification information has been received.

The specific information can be, for example, information for notifying that the user was near the second information processing device 200. This information may further include information of nearby facilities capable of dealing with infectious diseases, telephone numbers of consultation desks, and the like.

It is preferable to force the notification using push notification technology.

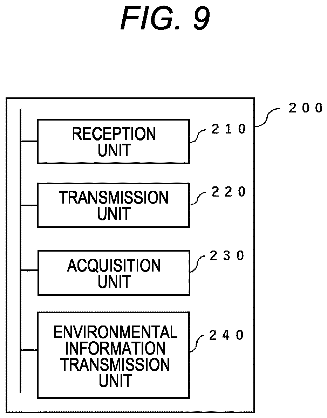

The second information processing device 200 can further include an acquisition unit 230 and an environmental information transmission unit 240 as shown in FIG. 9.

The acquisition unit 230 acquires environmental information of an installation location from a prescribed sensor.

Although the prescribed sensor is not particularly limited as long as it can measure the environmental information of the installation location, the prescribed sensor may be at least one of a room temperature sensor, a humidity sensor, an illuminance sensor, an atmospheric pressure sensor, a noise sensor, various types of gas concentration sensors, a discomfort index sensor, and a heatstroke alert level sensor.

Examples of gases that can be measured by the gas sensor can include CO₂ and volatile organic solvent lines.

The environmental information transmission unit 240 transmits time information about the time at which the environmental information was received to the server device 300 together with the environmental information.

The storage unit 310 of the server device 300 can store the environmental information and the time information received from the second information processing device 200 in association with the second identification information.

When the environmental information received from the second information processing device 200 satisfies a specific condition, the extraction unit 330 of the server device 300 may extract one or more pieces of first identification information stored in association with the second identification information.

The specific condition can be a condition that satisfies the so-called three Cs (closed, crowded, and close). Specific numerical values can be appropriately preset.

Also, the notification unit 340 described above can provide a notification of caution information to at least one of the first information processing devices 100 having one or more pieces of first identification information extracted when the specific condition is satisfied.

In other words, when the room satisfies the three Cs conditions, it is possible to notify the organizer of the conference or the like of caution information such as urging ventilation.

Alternatively, when the environmental information satisfies a specific condition, the three Cs may be eliminated by automatically controlling indoor loT devices (an air conditioner, an air purifier, a humidifier, and the like). Automated control of the loT device can be applied when the second information processing device 200 is an loT router, as will be described below.

The second information processing device 200 in the present disclosure can be an loT router.

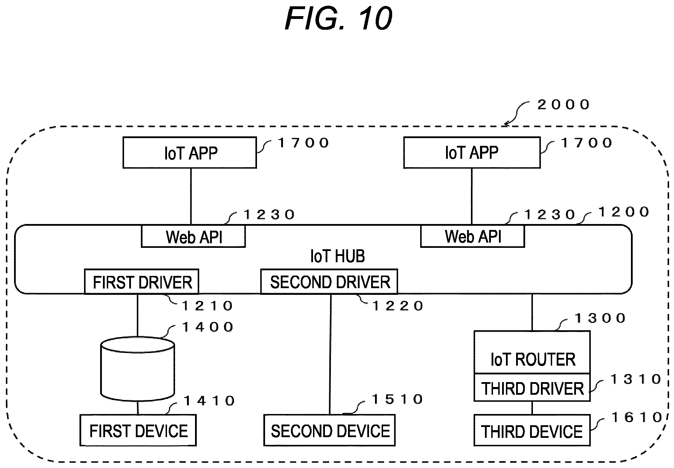

As shown in FIG. 10, the loT router is an loT router 1300 in an loT connection system 2000 including an loT hub 1200 implemented on the cloud and the loT router 1300 locally connected to the loT hub 1200.

Here, an embodiment of the loT connection system 2000 according to the embodiment of the present disclosure will be described with reference to the drawings.

As shown in FIG. 10, it is assumed that the loT connection system 2000 in the embodiment of the present disclosure includes the loT hub 1200 and the loT router 1300.

It is assumed that the loT hub 1200 is implemented on a cloud. Specifically, the loT hub 1200 is a managed service hosted in the cloud and functions as a repeater for two-way communication between an IoT application (hereafter referred to as an "loT app") and an loT device.

It is assumed that the loT router 1300 is locally located and connected to the loT hub 1200 by a wide area network (WAN).

Specifically, the loT router 1300 enables an loT device that is not connected to the Internet, such as a home network, to connect to the loT hub 1200.

The loT hub 1200 has at least one of the first driver 1210 and the second driver 1220.

The first driver 1210 and the second driver 1220 absorb differences in specifications of each loT device manufacturer.

The first driver 1210 is for connecting the private cloud 1400 to which the first device 1410 is connectable and the loT hub 1200.

As an example, the first device 1410 and the private cloud 1400 are preferably connected by a local area network (LAN) or a WAN and the private cloud 1400 and the first driver 1210 are also preferably connected by the LAN or the WAN.

The private cloud 1400 is provided by the business operator of the first device 1410. Although a case where there is one private cloud 1400 is shown in FIG. 10, the number of private clouds 1400 is not limited to one and a plurality of private clouds 1400 can be connected to the loT hub 1200. Also, the loT hub 1200 may have a plurality of first drivers 1210.

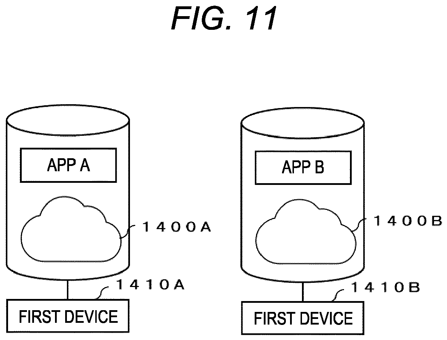

FIG. 11 shows details of two private clouds 1400A and 1400B provided by different business operators A and B. As shown in FIG. 11, the private cloud 1400A is connected to application A (hereinafter referred to as "app A") provided by business operator A and a service based on app A is provided to the first device 1410.

Likewise, the private cloud 1400B is connected to application B (hereinafter referred to as "app B") provided by business operator B and a service based on app B is provided to the first device 1410.

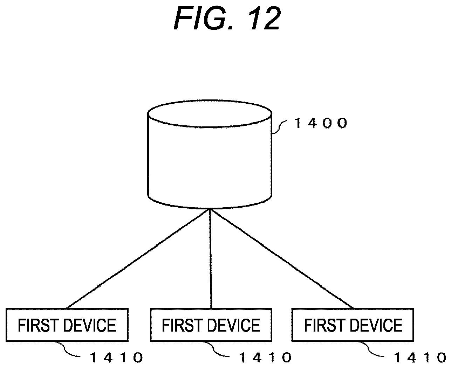

Although an example in which only one first device 1410 is connected to the private cloud 1400 is shown in FIGS. 10 and 11, a plurality of first devices 1410 may be connected to one private cloud 1400 as shown in FIG. 12.

The first device 1410 can be a device for which a business operator provides a private cloud. Examples of the first device 1410 include an electronic lock with a remote lock function, an artificial intelligence (Al) speaker, and a care bed that can be operated remotely, but are not particularly limited thereto.

Referring back to FIG. 10, the second driver 1220 is for directly connecting the second device 1510 and the loT hub 1200.

The second device 1510 can be connected to the loT hub 1200 on the Internet by the WAN (the second device 1510 may be connected to the loT hub 1200 via the LAN).

Although an example in which only one second device 1510 is connected to the second driver 1220 is shown in FIG. 10, a plurality of second devices 1510 may be connected to one second driver 1220. Also, the loT hub 1200 may have a plurality of second drivers 1220.

The second device 1510 can be a device for which the business operator does not provide a private cloud. Examples of the second device 1510 include fans, air conditioners, windows, curtains, and lighting, but are not particularly limited thereto.

The loT router 1300 has a third driver 1310. Also, the loT router 1300 may have a plurality of third drivers 1310.

The third driver 1310 is for connecting the third device 1610 and the loT router 1300.

As an example, it is preferable to connect the third device 1610 and the third driver 1310 by the LAN and connect the loT router 1300 and the loT hub 1200 by the WAN.

The third device 1610 can be an IoT device that is not connected to the Internet, such as a home network, as described above. Also, the third device 610 may be a device that should not be directly connected to the loT hub 1200 from the viewpoint of security, privacy, and safety. Examples of the third device 1610 include gas stoves, face authentication devices, data loggers for collecting sensor information, and the like, but are not particularly limited thereto. However, from the viewpoint of risk reduction in the event of a disaster, which will be described below, any device may be connected to the loT router 1300 as the third device 1610.

In this way, the loT connection system 2000 of the present invention does not directly connect all devices to the loT hub 1200 on the cloud, but is a hybrid type loT connection system for locally connecting some devices to the loT router 1300.

According to the above, it is possible to easily interconnect loT devices connected to a conventional private cloud as well as directly connected loT devices.

Thereby, unlike conventional loT devices of a certain manufacturer that were only connected to each other, IoT devices of various manufacturers can be easily interconnected. Also, it will be possible to create unique services that have never existed before by interconnecting loT devices from various manufacturers.

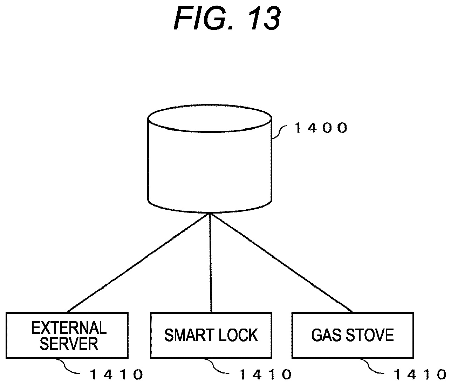

For example, according to the loT connection system 2000 of the present invention, as shown in FIG. 13, when an emergency earthquake early warning from an external server is received, services such as sending a fire extinguishing signal to a gas stove and unlocking a key of an entrance door can be easily implemented.

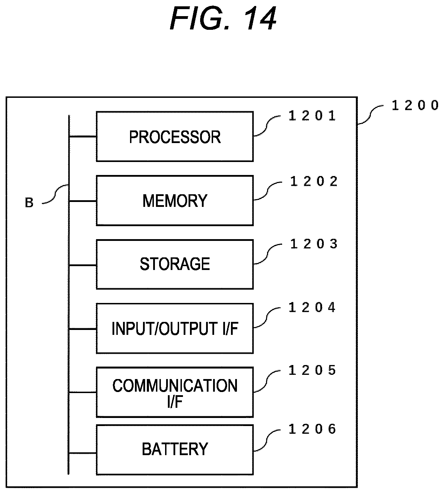

Here, the hardware configuration of the information processing terminal (IoT hub) 1200 will be described using FIG. 14. The information processing terminal 1200 can include a processor 1201, a memory 1202, a storage 1203, an input/output interface (input/output I/F) 1204, and a communication interface (communication I/F) 1205. The components are interconnected via a bus B.

The information processing terminal 1200 can implement the functions and methods described in the present embodiment through the cooperation of the processor 1201, the memory 1202, the storage 1203, the input/output I/F 1204, and the communication I/F 1205.

The processor 1201 executes functions and/or methods implemented by codes or instructions included in programs stored in the storage 1203. The processor 1201 may include, for example, a CPU, an MPU, a GPU, a microprocessor, a processor core, a multiprocessor, an ASIC, an FPGA, and the like and logic circuits (hardware) and dedicated circuits formed in integrated circuits (an IC chip and an LSI circuit) and the like may implement processes disclosed in the embodiments. Also, these circuits may be implemented by one or more integrated circuits and a plurality of processes shown in the embodiments may be implemented by one integrated circuit. Also, the LSI circuit may be referred to as a VLSI circuit, a super-LSI circuit, an ultra-LSI circuit, or the like according to a difference in a degree of integration.

The memory 1202 temporarily stores programs loaded from the storage 1203 and provides a work area for the processor 1201. The memory 1202 also temporarily stores various types of data generated while the processor 1201 is executing the program. The memory 1202 includes, for example, a RAM, a ROM, and the like.

The storage 1203 stores programs. The storage 1203 includes, for example, an HDD, an SSD, a flash memory, and the like.

The communication I/F 1205 is implemented as hardware such as a network adapter, communication software, or a combination thereof, and transmits and receives various types of data via a network. The communication may be performed by wire or wirelessly and any communication protocol may be used as long as mutual communication can be performed. The communication I/F 1205 communicates with other information processing devices via the network. The communication I/F 1205 transmits various types of data to other information processing devices in accordance with an instruction from the processor 1201. The communication I/F 1205 also receives various types of data transmitted from other information processing devices and transmits the data to the processor 1201.

The input/output I/F 1204 includes an input device for inputting various types of operations to the information processing terminal 1200 and an output device for outputting processing results after processes performed by the information processing terminal 1200. The input/output I/F 1304 may be integrated with the input device and the output device or may be separated into the input device and the output device.

The input device is implemented by any one or a combination of all types of devices that can receive an input from the user and transmit information related to the input to the processor 1201. The input device includes, for example, hardware keys of a touch panel, a touch display, and a keyboard, a pointing device such as a mouse, a camera (an operation input via an image), and a microphone (an operation input by voice).

The output device outputs a processing result of a process performed by the processor 1201. The output device includes, for example, a touch panel, a speaker, and the like.

The above-described loT router 1300 can also have a hardware configuration similar to that of the loT hub 1200.

Also, the loT router 1300 is preferably an information processing terminal capable of communicating with a prescribed base station.

An information processing terminal capable of communicating with a prescribed base station is, for example, an information processing terminal such as a smartphone or a tablet terminal into which a subscriber identification module (SIM) card is inserted for use. Because a communication capacity between the loT router 1300 and the loT hub 1200 is less than or equal to 100 Mbytes per month, it is sufficient to insert a low-cost SIM card with a small communication capacity into the loT router 1300. As an example, when there are an average of 10 beacons (persons) around one conference room and information is sent to the server every 5 minutes, the required communication capacity is 17.3 M/conference room (month).

Also, it is assumed that the loT router 1300 includes a battery. This battery can store electric power by performing a charging process. The loT router 1300 can implement the functions of the present disclosure without being connected to a power supply for about three days after being fully charged. It is assumed that the loT router 1300 is used while being connected to the power supply during normal times (when not under a specific situation to be described below).

The third device 1610 can be connected to the loT hub 1200 through a tethering function provided by the loT router 1300 under a specific situation.

The specific situation can be a situation in which the connection between the third device 1610 and the prescribed wireless/wired LAN is lost, a situation in which the connection between the loT router 1300 and the prescribed wireless/wired LAN is lost, or the like, but is not limited thereto.

Causes of this specific situation include, but are not limited to, power outages, and the specific situation may also be caused by Wi-Fi (registered trademark) router malfunctions, poor connections, or the like.

Also, known technology can be used as a tethering function, and methods such as Wi-Fi tethering, Bluetooth (registered trademark) tethering, and USB tethering can be used.

According to the above configuration, it is possible to provide attractive services by freely establishing an interconnection of the loT of daily life that is in a silo state for each private cloud via the Internet.

Specifically, according to the present disclosure, it is possible to easily interconnect IoT devices connected to a conventional private cloud as well as directly connected IoT devices.

Also, according to the above configuration, the third device 1610 is connected to the loT hub 1200 by the tethering function provided by the loT router 1300 under a specific situation, so that the service can be used continuously even if the loT device cannot be connected to the network in a disaster or the like.

Also, it is preferable that the loT router 1300 be capable of being remotely controlled by an administrator device. According to this configuration, each loT router can be fully supported.

Also, the above-described remote operation is implemented by installing a dedicated application on both an administrator device and the loT router 1300. Also, considering the ease of supporting a remote operation, it is preferable to use an Android terminal as the information processing terminal serving as the loT router 1300.

Also, the loT router 1300 is preferably subjected to connected device management (CDM) (management of various devices) by the administrator device. This CDM can be implemented using a known mobile device management (MDM) method. According to this configuration, loT routers installed in a huge number of households can be immediately and centrally managed and remotely monitored and updated. Also, the CDM enables remote monitoring and control of device settings, app distribution, and updates. Thereby, cost reduction and operational efficiency can be implemented.

Although only authenticated apps and devices can be connected to the IoT hub 1200, the loT router 1300 extends the loT hub 1200 locally (inside of the house or the like) and can be allowed to cope with the securement of real-time property, security, and privacy that can only be achieved locally, the reduction of an amount of communication, or the like.

Also, by using the loT router 1300 as an information processing terminal, there is no need to use a customer's smartphone or a home Internet circuit, and the risk of occurrence of unexpected trouble can be reduced.

Also, in the loT connection system 2000 of the present invention, the information to be written by the user to create the first driver 1210, the second driver 1220 and the third driver 1310 may be limited to information about a device definition and a command definition.

Here, a method of creating the first driver 1210, the second driver 1220, and the third driver 1310 will be described. Also, a creator can be a user involved in the manufacturing and development of IoT devices or a user involved in providing the loT connection system of the present invention.

Also, it is only necessary to describe the first driver 1210, the second driver 1220, and the third driver 1310 with the same information and programming languages may be different.

First, the creator defines a list of devices to be used in device definitions. For example, when "weather sensor," "indoor sensor," "outdoor sensor," "suspicious person sensor," "approval sensor," and "power sensor" are defined as devices to be used, these names and their IDs "weather," "inhouse," "outdoor," "security", "approve", and "power" are described.

Subsequently, the creator defines available commands in command definitions. As an example, when a usable command for "outdoor sensor" is defined, a sensor value observation command is described. Observation commands can be, for example, "observe," "get," "observe outdoors," and the like.

The first driver 1210, the second driver 1220, and the third driver 1310 can be completed by the creator filling in the information about the above device definitions and command definitions in the program in a fill-in-the-blank format. Other parts can be provided by the provider as a software development kit (SDK).

It is assumed that the SDK part includes a part related to a device operation process in a received command, a part related to preprocessing of collected sensor data/operation result data, and a part related to a process of data transmission to the IoT hub.

According to the above configuration, it is possible to simply complete drivers for various forms of IoT devices, so that it is possible to implement an IoT connection system that can flexibly and easily connect IoT devices.

Generally, engineers involved in device development have a field of acquired technology different from that of web development engineers and many do not have the technical level to connect devices to the IoT hub.

Thus, it is significantly beneficial to be able to create any driver program using a simple common fill-in-the-blank method as in the present disclosure. Thereby, the development cost and development period associated with the connection of the loT device to the loT hub can be limited more than before.

Also, due to the reduction in the development cost, even if there is a difference in the allowable cost of electric fans and air conditioners, it is possible to equally achieve the connection to the loT hub.

Next, the connection between the loT hub 1200 and the IoT app according to the loT connection system 2000 in the present disclosure will be described.

As shown in FIG. 10, the loT hub 1200 can have a web application programming interface (API) 1230 for utilizing an loT app 1700. Also, as shown in FIG. 10, the IoT apps 1700 can be connected in correspondence with the number of services and each loT app 1700 can be connected using the web API 1230.

The loT app 1700 is created by describing a data acquisition logic from the loT device (sensor) and/or an operation logic of the loT device within the app.

The data acquisition logic includes a part that pre-processes the acquired sensor data and a part that transmits the pre-processed data to an API of the loT hub 1200. Required information is a connection destination uniform resource locator (URL) provided from an operator of the loT connection system 2000 and an API key, device information, and an execution command provided from the operator.

The device operation logic includes a part that pre-processes the device command to be operated and a part that transmits the pre-processed device command to the API of the loT hub 1200. Required information is a connection destination URL provided from the operator of the loT connection system 2000 and an API key, device information, and an execution command provided from the operator.

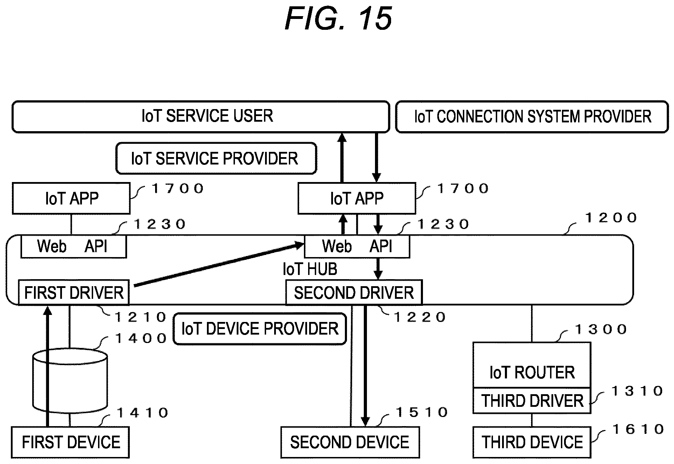

FIG. 15 is a diagram showing a flow in which an IoT service user (an end user) receives an IoT service provided from an IoT service provider using an IoT device provided by an IoT device provider. As shown in FIG. 15, when an event notification is first received from the first device 1410, the end user is notified of the event via the private cloud 1400, the first driver 1210, the web API 1230, and the IoT app 1700.

Subsequently, when the end user decides on an action, an instruction for the execution of the action is issued to the second device 510 via the loT app 1700, the web API 1230, and the second driver 1220.

The cooperation of loT devices can be represented by an event-driven program such as "If ~ happens like this, do OO." This process can be componentized as a microservice, shared and used and then implemented as a function of a function as a service (FaaS).

Meanwhile, the first to third devices that are the loT devices described above have been developed and manufactured by various manufacturers due to the recent development of the loT field. However, there is a problem that the constant connection with the device or loT app is limited to devices that can be connected to a server for a push notification service provided by a major vendor such as Google or Apple.

The loT hub 1200 according to the present disclosure implements a constant connection function and a directory function for the purpose of a combination of all loT devices.

The constant connection function allows the first device 1410, the second device 1510, the third device 1610, and the loT service (the loT app 1700) available via the loT hub 1200 to be connected to each other all the time.

However, two types of IoT devices connected to the loT hub include a type of IoT device whose constant connection is not essential and a type of IoT device whose constant connection is essential. The former includes a sensor-based device that periodically or irregularly transmits information. The latter includes a device such as a controllable device that needs to be remotely controlled. Consequently, it is only necessary for the above constant connection function in the present disclosure to implement a constant connection for the latter loT device that requires a constant connection and implement a connection when requested for the former IoT device that does not require a constant connection.

The constant connection function between the loT hub 1200, the first device 1410, the second device 1510, and the third device 1610, and the loT service 1700 in the present disclosure can be implemented using a communication protocol such as message queue telemetry transport (MOTT) for IoT.

Also, the directory function makes the first device 1410, the second device 1510, and the third device 1610 and the loT service cooperate with each other.

In other words, the directory function implements a function of identifying a thing or service from a certain thing (device) to a certain service, from a certain service to a certain thing, or from a certain thing to a certain thing, and giving an instruction for the thing or service.

In the directory function, it is possible to identify a cooperation destination device designated by a cooperation source device on the basis of device identification information, driver identification information, and connection interface identification information.

The device identification information is the UUID of the loT device. The driver identification information is an ID of the driver. The connection interface identification information is information for identifying an R-interface, which is the interface between the driver and the loT hub 1200.

Also, in the directory function, it is possible to further identify the device of the cooperation destination on the basis of the identification information of the loT hub 1200.

Also, device identification information, driver identification information, and connection interface identification information are stored in a prescribed storage device and the storage device can further store identification information of a connection source device that does not allow the connection and/or the function of the device as a whitelist. For such a list, it is possible to use a menu sheet of a support method to be described below.

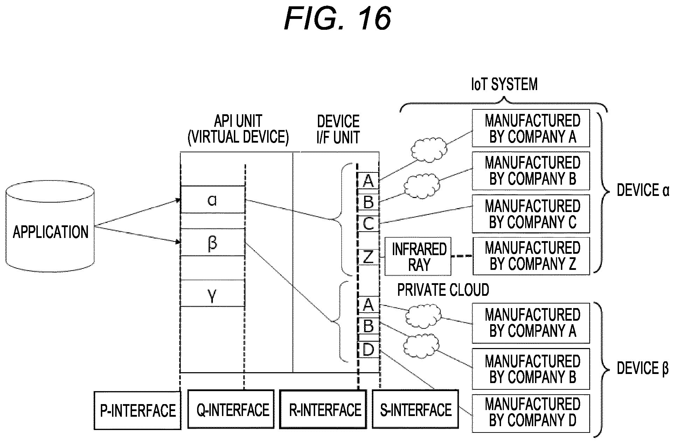

FIG. 16 is a conceptual diagram showing a structure of interconnection infrastructure in the loT connection system of the present disclosure. Although a connection surface between the IoT app shown as an application and the loT hub is a P-interface as shown in FIG. 16, it is possible to have a standardized Q-interface by establishing a connection using web APIs (α, β, and γ).

Likewise, the connection surface between the loT device and the loT hub is an S-interface, but it is possible to have a standardized R-interface by establishing a connection using drivers (A to Z).

In the related art, in order for the loT app to exchange information with an app embedded in the loT device, it is necessary to designate the loT device serving as a destination on the loT app side and it is also necessary to have identification information capable of being designated from the loT app on the loT device side.

On the other hand, in the loT connection system of the present disclosure, the designation of the above-described destination is performed using the directory function implemented by the loT hub.

For example, adding or replacing IoT devices can be handled simply by adding a driver to the loT hub and adding or switching the connection destination and there is no need to modify the loT app itself.

That is, a destination part that designates the loT device required in the loT app and an identification information part that can be designated from the loT app required in the loT device can be aggregated in any loT hub.

Thus, adding or replacing loT devices does not increase the man-hours of the app developer and leads to cost reduction.

Thus, according to the loT connection system of the present disclosure, even if the manufacturers and models of IoT devices are different, it is possible to implement a protocol-free interconnection by connecting to the IoT hub.

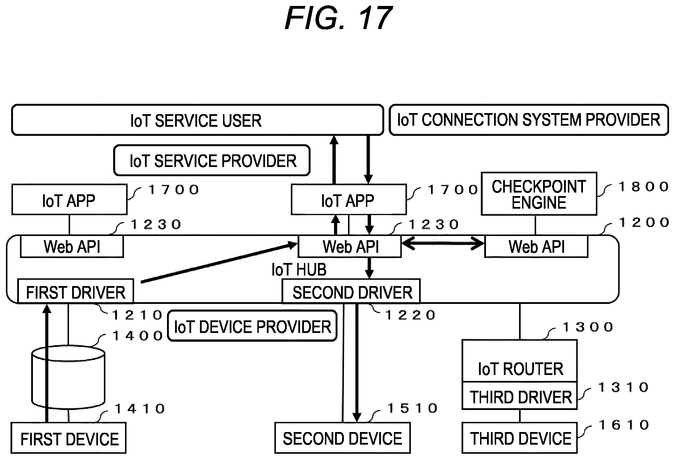

Also, as shown in FIG. 17, the loT hub 1200 of the present invention can be connected to a checkpoint engine 1800. This checkpoint engine 1800 checks content of an instruction issued to the device by the loT hub 1200 and prevents inappropriate actions from being executed.

For example, when an IoT service such as "If the outside air is fresh, turn off the air conditioner and open the window" is provided, the room may get wet if a torrential downpour hits immediately afterwards, but the checkpoint engine 1800 is for preventing such IoT-derived threats in advance.

Also, at least one of the first driver 1210, the second driver 1220, and the third driver 1310 of the present invention can implement a virtual device function.

In this virtual device function, the first device 1410, the second device 1510, or the third device 1610 is virtually reproduced.

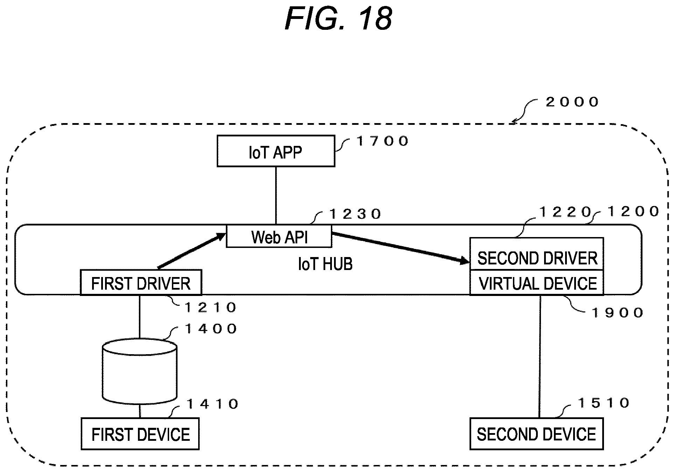

FIG. 18 shows an example in which the second driver 1220 of the present invention implements the virtual device function. As shown in FIG. 18, it becomes possible to develop an IoT app using the second device 1510 even if the second device 1510 is not connected by providing the virtual device 1900 capable of reproducing command transmission/reception of the second device 1510 in the second driver 1220. Also, if there is a malfunction, it is possible to easily isolate a failure by identifying which of the second device 1510 and the loT hub 1200 has failed without going to the site.

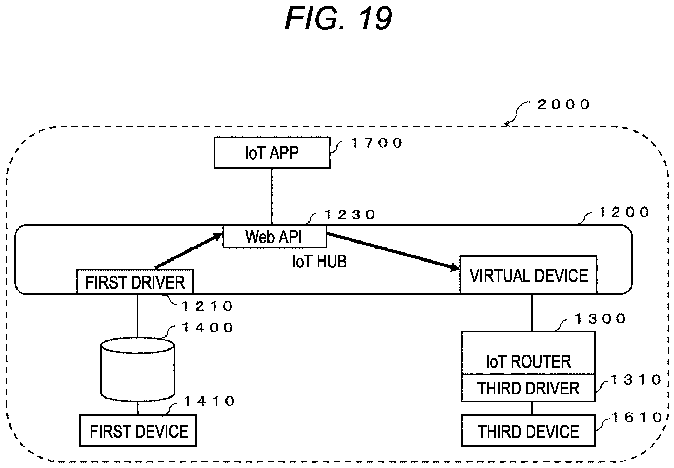

Also, in the present invention, as shown in FIG. 19, the loT hub 1200 instead of the driver may implement the virtual device function. This is an effective means for isolating the failure of the third device 1610 connected to the loT router 1300 that is located locally.

As described above, the loT hub in the present disclosure is a protocol-free infrastructure that also enables an interconnection between clouds using different communication protocols through interfaces called drivers. Also, the directory function makes it possible to combine and connect a plurality of apps and IoT devices in a complex way.

Also, in the loT connection system 2000 of the present invention, the information acquired from the first device 1410, the second device 1510, or the third device 1610 cannot be saved in the loT hub 1200.

It is assumed that the loT connection system 2000 of the present invention will be operated by a telecommunications carrier. Telecommunications carriers are obliged to comply with the confidentiality of communications, so they do not save information acquired from various types of devices for other purposes.

Because this information is useful information, it is normal for each company's private cloud to lock in the loT devices in order to acquire such information exclusively.

On the other hand, the loT connection system 2000 of the present invention can be operated by a telecommunications carrier and hence promote loT business by interconnecting an IoT device and an IoT app from a neutral standpoint.

Also, because the continuity of the loT interconnection service affects the continuity of the loT services of the companies using the loT interconnection service, it is preferable for the companies using the loT interconnection service to jointly share the interconnection infrastructure.

Also, in the loT connection system 2000 of the present invention, only devices and loT apps permitted by an API key and an authentication scheme can be connected to the loT hub 1200. That is, the loT connection system 2000 of the present invention can construct a closed network dedicated to loT communication on the Internet. Also, because a communication path is also encrypted and the loT router is also managed using a mobile device management (MDM) method, it is possible to cope with new attack methods and vulnerabilities of an operating system (OS) and an app.

Also, in order to connect a device that is not loT-enabled to the loT hub 1200, a backend as a service (BaaS) and an SDK can be utilized to perform development in a short period of time.

According to the above configuration, it is possible to provide a technical improvement for solving or alleviating at least some of the problems of the conventional technology described above. Also, according to the above configuration, it is possible to efficiently create a new service by combining the functions of a plurality of devices.

The server device 300 can further have a transmission unit 350 as shown in FIG. 8.

The transmission unit 350 transmits information about the unlock key capable of unlocking the specific electronic lock only to the first information processing device 100 corresponding to the first identification information received from the second information processing device 200.

In other words, it is possible to allow only people who have the app in their smartphones to enter and leave the conference room.

Although description has been given above on the assumption that the first information processing device 100 is a device such as a smartphone having a signal transmission function and is possessed by a user because the second information processing device 200 is a device having a function of receiving signals and is installed in a conference room or the like in a company in the above embodiment, these transmission and reception functions may be reversed. That is, the first information processing device 100 may have a function of receiving a signal and the second information processing device 200 may have a function of transmitting the signal.

Also, both the first information processing device 100 and the second information processing device 200 may have both the transmission function and the reception function. In this case, which device implements which function can be remotely and automatically switched on the basis of an instruction from the server device 300.

Also, the device for implementing the transmission function (the first information processing device 100 in the above-described embodiment) is not limited to the information processing device such as the smartphone described above and a tag-type transmitter (a so-called "beacon tag") can also be used.

According to this, it is possible to easily and reliably acquire a behavioral history of children and elderly people who do not/cannot have smartphones and medical and nursing care workers who cannot carry smartphones around and it is possible to easily and reliably identify those who were in the same place as the people described above.

Embodiments of services implemented by an information processing system, an information processing method, and a computer program according to the present disclosure will be described below.

### <Overview>

According to the service implemented by the information processing system, the information processing method, and the computer program in the present disclosure, an IoT sensor is installed in a conference room or the like and an employee who has entered the conference room for a certain period of time can be identified by the smartphone owned by the employee.

When an infected employee associated with a new coronavirus or the like is found among employees, the information can be used to identify close contacts. Thereby, it is possible to easily take a countermeasure for preventing the spread of infection such as having only those who have had close contact with the infected employee stay at home instead of all employees who have entered and exited the building.

In addition, by incorporating various sensors such as indoor environmental sensors as loT sensors, it is possible to take countermeasures such as continuously monitoring the indoor environment and preventing the spread of infection in the room as much as possible by appropriate ventilation or the like.

### <Providing method>

In providing this service, loT sensors and gateways (IoT routers) for enabling the sensors to communicate with the Internet will be provided. Among the loT sensors, a function for identifying employees entering the room by broadcasting a BLE beacon is built into the loT router.

Also, in order to identify that an employee entered the room after receiving a BLE beacon, it is necessary to install and register the application in the present disclosure in a company-owned or private smartphone in advance.

Room entry information and indoor environmental information collected by this mechanism are accumulated in the "Corona Tracer" service through the "loT Exchange" to be described in detail below and can be freely drawn by the person in charge of each company.

Also, an automation tool such as robotic process automation (RPA) may be used to retrieve information and take the action based on the information.

### <Service components and their functions>

In the "loT Exchange," commands for controlling sensors and information coming from the sensors are transmitted and received.

The "loT router" is responsible for connecting to the environmental sensor and transmits environmental data to the "loT Exchange." A built-in BLE chip plays a role as a BLE transceiver and can receive BLE beacons transmitted from the smartphone or transmit BLE beacons by itself.

The "environmental sensor" measures the environmental data (a room temperature, humidity, noise, an amount of CO₂, an amount of volatile organic solvent, or the like) of the conference room or the like and sends the measured environmental data to the "Corona Tracer" service via the loT router.

The "Corona Tracer App" supports iOS and Android, is installed on employees' smartphones (company-owned or personally-owned smartphones) and transmits a BLE beacon. Also, confirmation information for confirming that the BLE beacon is transmitted all the time is transmitted with respect to the "Corona Tracer" service. Also, a BLE beacon may be received and the information (a time stamp, a company ID, an employee ID, a beacon ID, or a beacon intensity) may be transmitted for the "Corona Tracer" service.

In the "Corona Tracer" service, information from environmental sensors, loT routers, and the "Corona Tracer App" is collected and saved. Also, in this service, a setup process for the loT router can be performed remotely. Also, when the loT router is used as a BLE transmitter, it is possible to change a quick output interval and a transmission intensity of BLE. The service can also generate a report for employee entry/exit information associated with the conference room and save the report in a csv file.

"RPA" or other systems that wish to cooperate access the "Corona Tracer" service, acquire information about employees entering and exiting the conference room and generate a report or transmit an alert based on the information.

### <Preparation for service provision>

In order to provide this service, the installation of the device in the conference room, the installation of the app and RPA by the employee, or various registration processes for other systems that wish to cooperate are necessary. Devices installed in the conference room are an IoT router, an environmental sensor, and an AC adapter. If there is no power supply in the room, a mobile battery is also required. The loT router is an Android smartphone, but kitting such as activation and application installation is performed by this service provider. Consequently, the installer only needs to place the set of devices in the conference room.

The apps to be installed on employees' smartphones are to be downloaded and installed by the employees from the Google Play Store or Apple App Store. Also, after installation, it is necessary to register a company ID and an employee ID.

Although the applications disclosed in the prior art literature are based on the assumption that both parties have apps installed on their smartphones, one party can operate with a tag or the like according to the invention of the present disclosure. Thereby, it is possible to support children and elderly people who do not have smartphones as well as medical and nursing care workers who cannot carry smartphones around.

Although the application disclosed in the prior art literature is only a method of saving in a smartphone, it is possible to select a method of saving in both the smartphone and the cloud according to the invention of the present disclosure. Also, the relative distance information of both parties and the information for identifying the person in question are separated as a completely separate system and a restriction is made so that access can only be made via a linkage system. Thereby, even if information on the terminal side and information on the cloud side can be accessed, it is impossible to obtain the information.

Although the application disclosed in the prior art literature is an application for smartphones and has no concept of a parent device or a child device and it is assumed that both terminals are equal and do not acquire location information, the mass infection location can be easily identified because the reception side can be installed in a specific place according to the invention in the present disclosure. Thereby, it is possible to easily identify the location and the date and time of occurrence of the mass infection. Specifically, there are conference rooms, toilets, shops, clubs, classrooms, and karaoke rooms. If the location and the date and time can be identified, it may be possible to provide a notification of the risk of infection even if the person in the place does not have a smartphone, an app, a tag, or the like. Also, according to the invention in the present disclosure, no matter how many child devices there are, because it is a method in which the parent device performs a reception process, the data accumulation/collection destination can be limited and unified. The location (including location information) of the parent device can be identified by the company by introducing and installing the parent device in a conference room or the like and the parent device is preferably operated and maintained remotely by the company.

Although this application is necessarily necessary for the application disclosed in the prior art literature and it is difficult to cooperate with other similar applications and services, systems in which a specific company, organization, and local government and the like are individually included can be easily interconnected and only information based on a contract can be shared with the party with whom the contract has been concluded according to the invention in the present disclosure. This ensures that information is not shared without the consent of individuals or contracts.

Although the application disclosed in the prior art literature is basically a general infection information sharing system that does not identify personal information as much as possible, the invention in the present disclosure can enable information about an installation location associated with a business operator to be added and hence it is possible to identify which location has become the infected location. As a result, the business operator can reduce the risk of business suspension and ensure the safety of their employees. Business operators described here are all business operators that have not been able to take individual countermeasures until now, such as companies (conference rooms and toilets), schools (classrooms and toilets), and shops (restaurants, karaoke, clubs, and the like).

Also, the invention in the present disclosure is linked with a global positioning system (GPS) function provided in the first information processing device and a mechanism in which a person's own location information can be submitted to prove that the person has been waiting at home for a long time when an infected person occurs in the vicinity of the person who has been ordered to stay at home after returning home may be constructed.

Finally, embodiments of the information processing method and computer program in the present disclosure will be described.

The information processing method according to the present disclosure is an information processing method executed in the information processing system 1000 including the first information processing device 100 having a function of transmitting a signal, the second information processing device 200 having a function of receiving the signal, and the server device 300 connectable to the second information processing device 200 via the Internet as shown in FIG. 1.

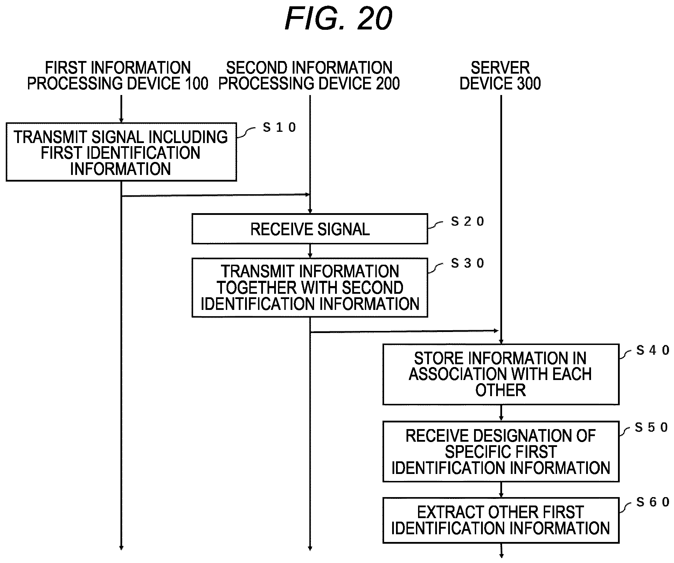

The information processing method according to the present disclosure causes the first information processing device 100 to execute step S10 of transmitting a signal including first identification information of the first information processing device 100, as shown in FIG. 20.

The information processing method according to the present disclosure causes the second information processing device 200 to execute step S20 of receiving the signal transmitted from the first information processing device 100 and step S30 of transmitting time information about the time at which the signal was received, intensity information based on a reception intensity of the signal, and the first identification information to the server device 300 together with second identification information of the second information processing device 200.

The information processing method according to the present disclosure causes the server device 300 to execute step S30 of storing the time information, the intensity information, the first identification information, and the second identification information received from the second information processing device 200 in association with each other; step S40 of receiving the designation of specific first identification information of a specific first information processing device 100; and step S50 of extracting one or more pieces of other first identification information stored in association with specific second identification information on the basis of the specific second identification information stored in association with the specific first identification information of the specific first information processing device 100 when the designation has been received.

As described above, according to the information processing method of the present disclosure, it is possible to easily and reliably acquire a behavioral history indicating when and where a person having a specific first information processing device was and it is possible to easily and reliably identify a person who was in the same place as the person. That is, the information processing method according to the present disclosure improves traceability of infected persons.

A computer program according to the present disclosure is a computer program to be executed in an information processing system including a first information processing device having a function of transmitting a signal, a second information processing device having a function of receiving the signal, and the server device capable of being connected to the second information processing device via the Internet.

The computer program according to the present disclosure causes the second information processing device to implement functions of: receiving a signal including first identification information of the first information processing device transmitted from the first information processing device; and transmitting time information about the time at which the signal was received, intensity information based on a reception intensity of the signal, and the first identification information to the server device together with second identification information of the second information processing device.

The computer program according to the present disclosure causes the server device to implement functions of: storing the time information, the intensity information, the first identification information, and the second identification information received from the second information processing device in association with each other; receiving the designation of specific first identification information of a specific first information processing device; and extracting one or more pieces of other first identification information stored in association with specific second identification information on the basis of the specific second identification information stored in association with the specific first identification information when the designation has been received.

As described above, according to the computer program of the present disclosure, it is possible to easily and reliably acquire a behavioral history indicating when and where a person having a specific first information processing device was and it is possible to easily and reliably identify a person who was in the same place as the person. That is, the computer program according to the present disclosure improves traceability of infected persons.

Finally, another application example of the information processing system according to the present disclosure will be described.

It has been reported that the new coronavirus is transmitted mainly through droplet contact with infected people, and public health agencies are calling for cutting off infection routes to control the spread of infection and for promptly identifying close contacts in the event of unavoidable contact and infection.

Thus, the government and infectious disease research institutes disclose places (facilities such as stores and business facilities) where infected people have been used and companies for providing services in which the places can be confirmed on a map using location information based on GPS and the like and services for tracking infection routes and providing a notification of close contacts have also appeared as in Non-Patent Literature 1.

In the service described in Non-Patent Literature 1, when smartphones on which the corresponding app is installed come close to each other, IDs assigned to the terminals are exchanged using "Bluetooth" of short-range wireless communication and recorded. When the infected person is an app user, an employee of the Ministry of Health will identify and contact a smartphone user who has been near the infected person for a certain period of time or more using the record on the app as a clue with the consent of the person in question after obtaining the person's consent and take necessary countermeasures.

By using such an app, it is possible to track close contacts who are not recognized by the person in question and it is effective for early detection of clusters (groups of infected people) and early isolation of people who are at risk of infection. However, it is assumed that this app is used on smartphones and the effect will not increase unless a large number of people use the app.

Currently, a state of emergency has been declared as a countermeasure to prevent the spread of the new coronavirus and many companies are being asked to refrain from going out. However, there are some types of work that cannot be handled at home or that are necessary to maintain social functions, and those who engage in such work are forced to continue to come to work.

Actually, at present, infected people have been found in companies, stores, hospitals, and the like that conduct work indispensable for ensuring the stability of people's lives and the national economy, and a situation in which not only specific work but also businesses themselves must be temporarily suspended has arisen.

For all business operators, a process of quickly identifying and sorting out close contacts has already become essential as a business continuity (BCP) countermeasure.

Therefore, the present inventor has developed a "close contact identification service" by utilizing the loT router 1300 in the present disclosure installed in a conference room or the like as the second information processing device 200 for the purpose of corporate BCP countermeasures.

First, the loT router (the parent device: the second information processing device 200) according to the present disclosure is installed in a place where the "three Cs" are likely to occur such as a corporate conference room. When an employee with a smartphone (the child device: the first information processing device 100) on which a beacon app is installed approaches there, the child device UUID of the employee and the distance are detected and recorded together with the time.

Thus, administrators in the general affairs department and the like can easily identify the names of employees who may have been in close contact simply by designating the names of infected employees.

This "close contact identification service" has a mechanism in which required data is acquired in the necessary amount when needed via the "loT hub" and "IoT router" in separate systems referred to as the "beacon data collection system" and the "close contact identification system" and has a mechanism in which the administrator cannot continue to monitor someone's behavior.

That is, the system including the first information processing device 100 and the second information processing device 200 in the present disclosure is the "beacon data collection system," the system including the server device 300 is the "close contact identification system" and the loT hub 1200 described above functions as a hub that connects these.

Thus, it is possible to achieve both high privacy protection and security. In other words, two systems including the "beacon data collection system," which limits data to be collected to only data that do not constitute personal information, and the "close contact identification system," which handles personal information such as employees' names and contact information, are created and information is blocked by separating these two systems.

By loosely coupling these systems using an loT hub only when necessary, it is possible to identify close contacts while preventing constant monitoring of employee behavior.

According to this system configuration, functions can be expanded by connecting systems with various functions to the loT hub 1200.

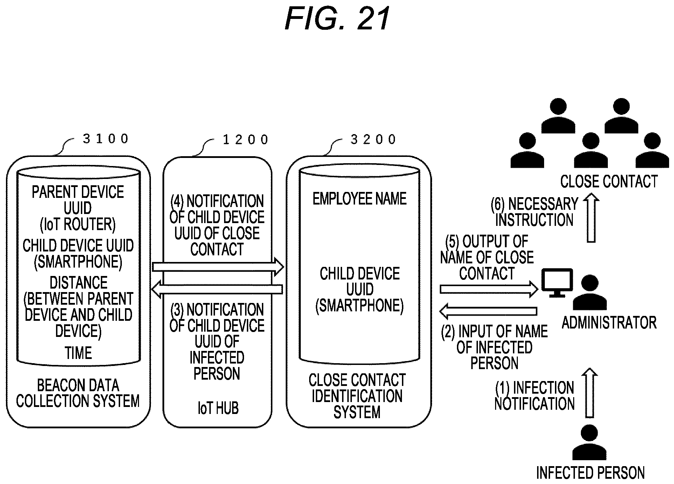

Next, a flow for identifying close contacts from the occurrence of infected people will be described using FIG. 21.

Specifically, data of the person in the conference room (a UUID, a time, and a distance between the loT router that is the parent device and the smartphone that is the child device) is automatically collected by using a beacon or a smartphone application installed in the conference room in advance in the beacon data collection system 3100 shown in FIG. 21. The beacon data collection system 3100 does not include personal information

When an administrator has received an infection report from an infected employee, the administrator inputs an employee's name from a management screen. The close contact identification system 3200 sends an infected person's UUID to the beacon data collection system 3100. On the basis of this UUID, the beacon data collection system 3100 identifies the UUID at risk of close contact and sends the identified UUID to the close contact identification system 3200. The system 3200 associates UUIDs with names and outputs an association result as a close contact candidate list. A person in charge of general affairs, who is the administrator, assigns priority levels from information of a list, contacts candidates for the close contact, and instructs the candidates to stay at home, conduct tests, and continue reporting.

In "Corona Tracer," BLE is used as a beacon. It is also possible to detect close contact by utilizing the beacon between smartphones. However, considering that there are employees who do not have smartphones and some smartphones are not capable of two-way communication of BLE, the loT router is used as the parent device and employees who cannot communicate with BLE in both directions have the beacon tag so that all employees can use the system. Data collected by using BLE between smartphones is used for a supplementary purpose such as an improvement of distance accuracy.

The administrator of this system will notify the employee of an ID and password by e-mail. When the employee who has received the notification input the ID and the password sent when installing the app, a UUID is automatically generated in the smartphone and registered with the close contact identification system along with the name. Because only this UUID is sent to the beacon data collection system and used for recording, an individual cannot be identified from the UUID.

Also, data of different companies can be shared on a limited basis based on the contract using the loT hub. When this function is utilized, it is possible to inform employees of another company in the same room that they are at risk of infection.

Also, if the introducing company agrees, it is possible to hide personal information and provide information collectively to the government, local governments, or insurance institutions. Thereby, it becomes possible to quickly provide information about infected clusters.

Finally, an example using a medium on which a two-dimensional code is printed instead of or in addition to the first information processing device 100 will be added.

Although description has been given on the assumption that a target person is a person who has the first information processing device 100 on which a dedicated application is installed in the above-described embodiment, a person who has another information processing device (an information processing terminal on which a dedicated application is not installed), a person who does not have an information processing terminal, or the like may visit the company in many cases.

For example, for a visitor such as a worker who performs construction work or the like or a person who temporarily visits for a conference or the like, a process of printing a two-dimensional code such as a QR code (registered trademark) on an admission card and managing information contained in this two-dimensional code is easier than a process of performing management using a beacon tag or a smartphone on which the above-described dedicated application is installed.

This QR code records a visitor's name and contact information (email address and/or phone number and the like). The information may be input by the visitor via a prescribed input terminal when visiting or may be registered in advance by the visitor or a person who is to welcome the visitor.

When the visitor enters the conference room, the visitor causes the second information processing device 200, which is the parent device installed in the conference room, to read a two-dimensional code printed on a visitor's admission card. Thereby, information of the visitor and information such as the read time are recorded in the server device 300.

Also, the two-dimensional code is not limited to being read by the parent device, and may be read by the first information processing device 100, which is a child device, and transmitted to the parent device.

In combination with the above-described embodiment, when it is necessary to contact a visitor, the contact is preferably performed by sending an e-mail or short message service (SMS) message to the e-mail address or telephone number recorded as a contact destination.

Further, the above two-dimensional code can also be used as an online business card. In other words, instead of exchanging business cards in the conventional way of exchanging actual paper media, the content of business cards is recorded in a two-dimensional code and displayed on a screen of a smartphone or the like, thereby avoiding physical contact and exchanging business cards remotely.

Also, by displaying online business cards on the screen shared by the participants at the conference, it is possible to exchange business cards with the participants all at once.

While several embodiments of the present invention have been described above, these embodiments have been presented by way of example only, and are not intended to limit the scope of the inventions. These embodiments may be embodied in a variety of other forms. Various omissions, substitutions, and combinations may be made without departing from the spirit of the inventions. The inventions described in the accompanying claims and their equivalents are intended to cover such embodiments or modifications as would fall within the scope and spirit of the inventions.

Also, the method described in the embodiment can be stored as a program capable of being executed by a computer in a recording medium, for example, such as a magnetic disk (a floppy (registered trademark) disk, a hard disk, or the like), an optical disc (a compact disc (CD)-ROM, a digital versatile disc (DVD), a magneto-optical disc (MO), or the like), or a semiconductor memory (a ROM, a RAM, a flash memory, or the like), or can be transmitted and distributed via a communication medium. The program stored in the medium also include a setup program for configuring software means (including not only execution programs but also tables and data structures) to be executed by the computer within the computer. A computer that implements this device reads a program recorded on a recording medium, and constructs software means by a setup program in some cases, and executes the above-described process by controlling the operation using the software means. The term "recording medium" described here is not limited to those for distribution, and includes storage media such as magnetic disks and semiconductor memories provided in computers or devices connected via a network. The storage unit may function, for example, as a main storage device, an auxiliary storage device, or a cache memory.

### [LIST OF REFERENCE NUMERALS]

- 1000: Information processing system
- 100: First information processing device
- 110: Transmission unit
- 120: Acquisition unit
- 130: Environmental information transmission unit
- 200: Second information processing device
- 210: Reception unit
- 220: Transmission unit
- 300: Server device
- 310: Storage unit
- 320: Reception unit
- 330: Extraction unit
- 340: Notification unit
- 350: Transmission unit

## Claims

1. An information processing system including a first information processing device having a function of transmitting a signal, a second information processing device having a function of receiving the signal and installed at a specific location, and a server device capable of being connected to the second information processing device via the Internet,
wherein the first information processing device transmits a signal including first identification information of the first information processing device,
wherein the second information processing device receives the signal transmitted from the first information processing device and transmits time information about the time at which the signal was received, intensity information based on a reception intensity of the signal, and the first identification information to the server device together with second identification information of the second information processing device,
wherein the server device stores the time information, the intensity information, the first identification information, and the second identification information received from the second information processing device in association with each other, and
wherein the server device extracts one or more pieces of other first identification information stored in association with specific second identification information on the basis of the specific second identification information stored in association with specific first identification information when the designation of the specific first identification information of a specific first information processing device has been received.

2. The information processing system according to claim 1, wherein the server device extracts one or more pieces of other first identification information stored in association with time information associated with specific time information on the basis of the specific time information about a specific time at which a signal including the specific second identification information stored in association with the specific first identification information and the specific first identification information was received when the designation of the specific first identification information has been received.

3. The information processing system according to claim 1 or 2, wherein the server device provides a notification of specific information to the first information processing device having the one or more pieces of the first identification information extracted when the designation of the specific first identification information has been received.

4. The information processing system according to claim 1, 2, or 3,
wherein the second information processing device is connected to the server device via the Internet, acquires environmental information of an installation location from a prescribed sensor, and transmits time information about the time at which the environmental information was received to the server device together with the environmental information, and
wherein the server device stores the environmental information and the time information received from the second information processing device in association with the second identification information.

5. The information processing system according to claim 4, wherein the server device extracts one or more pieces of the first identification information stored in association with the second identification information when the environmental information received from the second information processing device satisfies a specific condition.

6. The information processing system according to claim 5, wherein the server device provides a notification of caution information to at least one of first information processing devices having the one or more pieces of the first identification information extracted when the specific condition is satisfied.

7. The information processing system according to any one of claims 1 to 6,
wherein the second information processing device is an Internet of Things (IoT) router in an IoT connection system including an IoT hub implemented on a cloud and the loT router locally connected to the loT hub,
wherein the loT hub has at least one of a first driver for connecting a private cloud to which a first device is connectable and the loT hub and a second driver for connecting a second device and the loT hub, and
wherein the loT router has a third driver for connecting a third device and the loT router.

8. The information processing system according to any one of claims 1 to 7, wherein the server device transmits information about an unlock key capable of unlocking a specific electronic lock only to the first information processing device corresponding to the first identification information received from the second information processing device.

9. The information processing system according to any one of claims 1 to 8, wherein the first information processing device includes a smartphone and/or a beacon tag.

10. The information processing system according to any one of claims 1 to 9, further comprising a medium on which a two-dimensional code in which third identification information is recorded is represented,
wherein the second information processing device reads the third identification information from the medium and transmits time information about the time at which the third identification information was read and the third identification information to the server device together with the second identification information of the second information processing device.

11. The information processing system according to any one of claims 1 to 10, wherein the server device can switch a function between a function of transmitting a signal of the first information processing device and a function of receiving the signal of the second information processing device.

12. An information processing method to be executed by an information processing system including a first information processing device having a function of transmitting a signal, a second information processing device having a function of receiving the signal and installed at a specific location, and a server device capable of being connected to the second information processing device via the Internet, the information processing method comprising steps of:
transmitting, by the first information processing device, a signal including first identification information of the first information processing device;
receiving, by the second information processing device, the signal transmitted from the first information processing device;
transmitting, by the second information processing device, time information about the time at which the signal was received, intensity information based on a reception intensity of the signal, and the first identification information to the server device together with second identification information of the second information processing device;
storing, by the server device, the time information, the intensity information, the first identification information, and the second identification information received from the second information processing device in association with each other;
receiving, by the server device, the designation of specific first identification information of a specific first information processing device; and
extracting, by the server device, one or more pieces of other first identification information stored in association with specific second identification information on the basis of the specific second identification information stored in association with the specific first identification information when the designation has been received.

13. A computer program to be executed in a server device of an information processing system including a first information processing device having a function of transmitting a signal, a second information processing device having a function of receiving the signal and installed at a specific location, and the server device capable of being connected to the second information processing device via the Internet,
wherein the second information processing device receives a signal including first identification information of the first information processing device transmitted from the first information processing device and transmits time information about the time at which the signal was received, intensity information based on a reception intensity of the signal, and the first identification information to the server device together with second identification information of the second information processing device, and
wherein the computer program causes the server device to implement functions of:
storing the time information, the intensity information, the first identification information, and the second identification information received from the second information processing device in association with each other; and
extracting one or more pieces of other first identification information stored in association with specific second identification information on the basis of the specific second identification information stored in association with specific first identification information when the designation of the specific first identification information of a specific first information processing device has been received.
